(19) Europäisches Patentamt
European Patent Office
Office européen des brevets

(11) Publication number: 0 287 916
A1

# (12) EUROPEAN PATENT APPLICATION

(21) Application number: 88105721.0

(22) Date of filing: 11.04.88

(51) Int. Cl.4: G01N 33/574, G01N 33/577, G01N 33/531

(30) Priority: 13.04.87 JP 91678/87

(43) Date of publication of application:
26.10.88 Bulletin 88/43

(84) Designated Contracting States:
CH DE ES FR GB IT LI NL SE

(71) Applicant: OTSUKA PHARMACEUTICAL CO., LTD.
9, Kandatsukasacho 2-chome
Chiyoda-ku Tokyo 101(JP)

(72) Inventor: Tachikawa, Tetsuya 13-6,
Aza-Higasinogamimoto
Takabo Kitajima-cho
Itano-gun Tokushima-ken(JP)
Inventor: Noda, Atsunari
463-10, Kagasuno Kawauchi-cho
Tokushima-shi Tokushima-ken(JP)
Inventor: Shin, Sadahito 40-20, Aza-Nakazao
Shinkirai Kitajima-cho
Itano-gun Tokushima-ken(JP)

(74) Representative: Hansen, Bernd, Dr.rer.nat. et al
Hoffmann, Eitle & Partner Patentanwälte
Arabellastrasse 4
D-8000 München 81(DE)

## (54) Immunoassay.

(57) Disclosed is an immunoassay for determining Disialosyl Le$^a$ antigen, characterized by using an acid-soluble fraction of excrement as the antigen in the determination system.

EP 0 287 916 A1

## IMMUNOASSAY

This invention relates to an immunoassay, and more particularly to a novel immunoassay for determining Disialosyl Le$^a$ antigen which is useful for screening and diagnosing cancers.

Disialosyl Le$^a$ antigen is a cancer antigen of carbohydrate nature which is known to be useful as a serum tumor marker. E. Nudelman et al discovered that this antigen is identical with a glycolipid in respect of the carbohydrate linkage having the structure represented by the following formula (1), the glycolipid being termed $III^4FucIII^6NeuAcIV^3NeuAcLC_4$ and separated from colonic adenocarcinoma:

```
                      NeuAcα2
                            ↓
                            6
NeuAcα2→3Galβ1→3GlcNAcβ1→3Galβ1→4Glcβ1→4Cer        (1)
                            4
                            ↑
                        Fucα1
```

The carbohydrates, lipids, modes of combination thereof, etc. are herein expressed in terms according to IUPAC-IUB and generally used in the art or in terms which are conventional, as exemplified below.

NeuAc: N-acetylneuraminic acid
Gal: galactose
Glc: glucose
GlcNAc: N-acetylglucosamine
Fuc: fucose
Cer: ceramide

The specific antibody recognizing the Disialosyl Le$^a$ antigen has been established [e.g. Monoclonal antibody termed "FH-7" by E. Nudelman et al is known. The monoclonal antibody FH-7 is known to be reactive also with monosialosyl Le$^a$ II ($III^4FucIII^6NeuAcLc_4$). The "Disialosyl Le$^a$ antigen" referred to herein is intended to include the carbohydrate linkage of such antigen.] The method of determining said antigen by an immunoassay using the above antibody has been reported.

Generally when a specific antibody has been established, the antigen recognized by the antibody can be determined by an immunoassay. Determination systems have been engineered and established in respect of various antigens. However, while the antibody recognizing the Disialosyl Le$^a$ antigen has been established, the immunoassays heretofore reported have posed the serious problem over the preparation of an antigen in the determination system. With the problem unresolved, a satisfactory method for determining the Disialosyl Le$^a$ antigen has not been heretofore developed. Although most basically it is possible to assume the use of the glycolipid having the carbohydrate linkage structure of the formula (1) as the antigen in the determination system, the purification of the glycolipid involves numerous and complex procedures as reported by E. Nudelman et al and consequently produces such a small amount of contemplated glycolipid as to permit only a slight degree of structure determination. With this drawback, the glycolipid can not provide the antigen useful in the determination technique. Further because of its further problems on the conditions of determination system, e.g. being insoluble in water, the glycolipid can not meet the demands for antigens sought after in the art.

Fukushi et al proposed a determining method in which a crude extract fraction of Disialosyl Le$^a$ antigen produced from a cancer cell line expressing Disialosyl Le$^a$ antigen is used as an antigen in the determination system in place of the glycolipid (purified antigen) with the carbohydrate linkage structure of the formula (1). Since the cancer cell line is difficult to mass culture and Disialosyl Le$^a$ antigen is difficult to extract from the cancer cell line (Disialosyl Le$^a$ antigen is not released into the culture medium by incubation), the antigen to be used in this method can be prepared only in small quantities and thus is not satisfactory for practical use.

These prior art techniques are disclosed in, for example, the following publications:

(i) Edward Nudelman et al, "The Journal of Biological Chemistry," Vol. 261, No. 12, pp 5487-5495 (1986);

(ii) Yasuo Fukushi et al, Proceedings of the Japanese Society for Immunology, Vol. 16, p 641, published by the Japanese Society for Immunology on Nov. 15, 1986; and

(iii) Reiji Kannagi, "Clinical Pathology," Vol. 34, No. 11, pp 1247-1264 (1986).

In the current situation, it is desired in the art to develop a technique for determining Disialosyl $Le^a$ antigen by an immunoassay which can be easily conducted with high sensitivity and high precision through simplified procedures.

An object of the present invention is to provide an immunoassay for determining Disialosyl $Le^a$ antigen which can overcome these technological problems and which can be easily carried out with high sensitivity and high precision through simplified procedures.

Another object of the invention is to provide a novel antigen in the determination system for the determination technique in the foregoing object.

According to the present invention, there are provided an acid-soluble fraction of excrement as Disialosyl $Le^a$ antigen and an immunoassay for determining Disialosyl $Le^a$ antigen, characterized by using the acid-soluble fraction of excrement as the antigen in the determination system.

We conducted extensive research and discovered that excrement contains Disialosyl $Le^a$ antigen and that surprisingly the content of the antigen therein is substantial to great advantage for the production of the antigen. We further found that the antigen can be easily prepared as an acid-soluble fraction of excrement and that the acid-soluble fraction of excrement (hereinafter referred to as "antigen of the invention") can be used without producing adverse effect in the immunoassay for determining Disialosyl $Le^a$ antigen. We confirmed, moreover, that the desired determination result can be obtained using this antigen with unexpectedly high precision and high sensitivity.

The present invention has been accomplished based on these novel findings. The novel immunoassay of the present invention is particularly useful for screening and diagnosing cancers.

We will describe below in detail a process for preparing the antigen of the invention.

The antigen of the invention can be easily prepared by subjecting the excrement of humans or other animals to extraction with an acid. While the kind of excrement useful as the starting material is not specifically limited, the excrement of humans, particularly meconium or fetuses is among preferred examples. The extraction with an acid can be carried out by various methods heretofore commonly employed. For example, an acid is added to excrement to a final concentration of about 0.4 M to about 1.2 M, preferably about 0.6 M, and the supernatant of the mixture is collected by centrifugation or like method. Examples of useful acids are hydrochloric acid and like mineral acids, acetic acid, citric acid, perchloric acid (PCA) and like organic acids among which PCA is preferred.

The resulting acid-soluble fraction of excrement as it is can be used as the antigen of the present invention. When required, the fraction is purified by gel filtration, dialysis or like usual purification procedure for removal of the acid from the fraction to provide the antigen of the invention.

The immunological determination method of this invention for determining Disialosyl $Le^a$ antigen critically uses the antigen of the invention thus obtained in the determination system and basically include common immunoassays such as radioimmunoassay (RIA), enzyme immunoassay (EIA), etc. These methods can be practiced by procedures and means which are generally employed, such as competition method, sandwich method and the like.

Body fluids usable as samples in the immunoassay of the invention, for example, for screening and diagnosing cancers are, for example, blood, cell fluid, lymph, pleural fluid, peritoneal fluid, amniotic fluid, gastric juice, urine, pancreatic juice, spinal fluid, saliva, etc. Among these, blood, especially serum or blood plasma is desirable.

The antibody to be used in the immunoassay of the invention is not specifically limited insofar as it is able to recognize Disialosyl $Le^a$ antigen. Typical examples of the antibody are the monoclonal antibody FH-7 as described above and other antibodies produced by conventional methods [Method in Enzymology, Vol. 73, p 3 (1981), etc.].

The immunoassay of the invention can be performed also by known solid phase techniques. In this case, the antigen of the invention or antibody is insolubilized by being caused to chemically or physically react with an insoluble carrier in the usual manner. Examples of useful insoluble carriers are cellulose powder, Sephadex, Sepharose, polystyrene, filter paper, carboxymethylcellulose, ion exchange resin, dextran, plastics film, plastics tube, nylon, glass beads, silk, polyamine-methyl vinyl ether-maleic acid copolymer, amino acid copolymer, ethylene-maleic acid copolymer and the like. The insolubilization is accomplished by the diazo method as a covalent method, peptide method (with use of an acid amide derivative, carboxy chloride resin, carbodiimide resin, maleic anhydride derivative, isocyanate derivative, cyan bromide-activated polysaccharide, cellulose carbonate derivative, condensation reagent or the like), alkylation method, carrier bonding method using a crosslinking reagent (using glutaraldehyde, hexamethylene isocyanate or the like as the crosslinking reagent), carrier binding method resorting to Ugi reaction and like chemical reaction methods; the ion bonding method using a carrier such as an ion exchange resin; and the physical adsorption method wherein glass beads or like porous glass is used as a carrier. When the agglutination method is used, the antibody is used as adsorbed by (sensitized with) particles, such as erythrocytes or latex particles, which are usually used for the agglutination test.

For labeling the antigen of the invention or antibody in the above determination methods, various common labeling agents are used which include radioactive substances, enzymatic labeling substances and fluorescent substances. Examples of useful labeling agents are radioactive substances such as $^{125}I$ or like radioactive iodine, fluorescent substances such as fluorescein isothiocyanate (FITC), tetramethylrhodamine isothiocyanate (TRITC), substituted-rhodamine isothiocyanate (XRITC), Rhodamine B isothiocyanate, dichlorotriazinefluorescein (DTAF) and the like, and enzymatic labeling substances such as peroxidase (POX), mycloperoxidase, chymotryspsinogen, procarboxypeptidase, glyceroaldehyde-3-phosphate dehydrogenase, amylase, phosphorylase, deoxyribonuclease (DNA-ase), ribonuclease (RNA-ase), alkali phosphatase, $\beta$-galactosidase and the like. Such labeling agents can be applied in the usual manner [see J. Biol. Chem., 254, 9349-9351 (1979); Nature, 194, 495 (1962); "Fluorescent Antibody Technique," Medical Chemistry Experiment Lecture No.4, 263-270; Acta. Endocrinol Suppl., 168, 206 (1972): Proc. Nat. Acad. Sci., USA.57, 713 (1967); etc.]

The reaction of the determination system can be conducted in the same manner as usual immunoreactions of this type. The solvent to be used in the determination system is a usual one which will not adversely affect the reaction. Examples of preferred solvents are buffers having a pH of about 4 to about 8 such as citrate buffer, phosphate buffer, Tris-HCl buffer, acetate buffer and the like. The conditions for immunoreaction in determination are not limited specifically; those usually employed for determination methods of this type are useful. More specifically, the immunoreaction is conducted generally at a temperature of up to about 45°C, preferably at about 4 to about 40°C, for about 1 to about 40 hours. After the reaction, the combined product (B) is separated from the free substance (F) by a known method. When the solid phase method is resorted to, the solid phase can be separated from the liquid phase, for example, by centrifuging, filtration, washing or decantation. In other cases, the dextran-coated charcoal method, double antibody method or like conventional method is used.

The antigen of the invention is used preferably as an insolubilized antigen and/or as a standard antigen in the immunoassay of the invention.

A preferred method using the antigen of the invention as an insolubilized one will be specifically described below. This method, competitive inhibition assay, comprises the steps of: performing competitive reaction in which the substance to be determined in the sample and a specific amount of insolubilized antigen are reacted respectively with specific amounts of labeled antibody; separating the solid phase from the liquid phase; and checking the labeling reagent for the activity to determine quantitatively the mass of the substance.

The immunoassay of the invention can be performed very conveniently using an assay kit incorporating the antigen of the invention as the essential component. The antigen reagent of the kit containing the antigen of the invention may have incorporated therein a stabilizer, such as glycerol or bovine serum protein, and/or a preservative. The kit may contain an antibody reagent containing the specific antibody recognizing Disialosyl $Le^a$ antigen. A buffer for holding the reagent system at a predetermined pH after reconstitution and/or a preservative and/or a stabilizer for preventing the degradation of sample before use can be further incorporated in the antibody reagent. Although the buffer is not considered to be an essential component of the kit reagent, it is desirable to add such a substance as to give a pH of about 4 to about 8 to the system when this method is practiced. While the reconstituting agent is preferably one containing water, the water can be partially or wholly replaced by a solvent which is miscible with water. Solvents which are miscible with water are well known to those skilled in the art. Examples of useful solvents are glycerins, alcohols, glycols, glycol ethers, etc.

According to the present invention, there is provided an immunoassay for determining Disialosyl $Le^a$ antigen having improved properties particularly for diagnosis of cancers. The immunoassay of the invention can conveniently determine Disialosyl $Le^a$ antigen with great ease, high sensitivity and high accuracy. Consequently the immunoassay of the invention is very useful particularly for screening and diagnosing cancers.

The present invention will be described in greater detail with reference to the following examples, reference examples in which the drawings referred to are :

Fig. 1 is a graph showing the result obtained by counting the radioactivity of labeled antibody in the immunoassay using the antigen of the invention;

Fig. 2 is a graph showing the result (standard curve) obtained by carrying out the immunoassay of Example 1 illustrating the invention; and

Fig. 3 is a graph showing the result (standard curve) obtained by performing the immunoassay of Example 2 illustrative of the invention.

Fig. 4 is a graph showing the result (standard curve) obtained by performing the immunoassay of Example 4 illustrative of the invention.

Reference Example 1

Preparation of antigens

(1) To 50 g of human meconium was added 100 ml of 0.6M PCA aqueous solution, and the mixture was stirred and centrifuged (8,000 × g, 15 minutes), giving a supernatant. The supernatant thus obtained was neutralized with a 10N NaOH aqueous solution and dialyzed against distilled water for 1 day to remove the PCA. Lyophilization gave the desired acid-soluble fraction (antigen of the invention).

(2) An antigen according to the invention was prepared from the excrement of a healthy adult by carrying out the same procedure as in (1).

Reference Example 2

Preparation of insolubilized antigens

Ten thousand polystyrene beads (4 mm in diameter, product of Sekisui Chemical Co., Ltd., Japan) were thoroughly washed with a 1/1000N NaOH aqueous solution containing 30% ethanol and further with distilled water.

Each of the antigens obtained in Reference Example 1-(1) and -(2) was dissolved in 0.1M bicarbonate buffer to a concentration of 20 μg/ml. To 60 ml of the solution were added 400 of the beads treated above. The mixture was stirred for 2 hours and left to stand at 4°C overnight. The beads were filtered off and washed with physiological saline. The beads were stirred in 50 mM PBS (pH 7.4) containing 0.5% crystal BSA (bovine serum albumin; product of Seikagaku Kogyo Co., Ltd., Japan) for 2 hours and left to stand at 4°C overnight. The beads were filtered off and thoroughly washed to obtain an insolubilized antigen. In this way, two types of insolubilized antigens were prepared.

The insolubilized antigen prepared from the antigen resulting from the meconium in Reference Example 1-(1) is abbreviated to "NCA", and the insolubilized antigen obtained from the antigen given in Reference Example 1-(2) to "NFA".

Reference Example 3

Preparation of standard antigens

Each of the antigens obtained in Reference Example 1-(1) and -(2) was diluted with sheep serum to a concentration of 100 ng/ml. This solution was further diluted to give solutions at varying concentrations of from 224 U/ml to 7 U/ml. The solutions are hereinafter referred to as "standard antigen".

The standard antigen obtained from the meconium-derived antigen afforded in Reference Example 1-(1) is hereinafter called "NCA standard antigen", and the standard antigen from the antigen given in Reference Example 1-(2) "NFA standard antigen".

Reference Example 4

Preparation of insolubilized antibody

A monoclonal antibody FH-7 [J.B.C., 261 (12), pp 5487-5495 (1986)] was adjusted to a concentration of 20 µg protein/ml with 50 mM PBS (pH 7.4) containing 0.15M NaCl and 0.05% $NaN_3$.

From 100 ml of the antibody solution thus obtained was prepared an insolubilized antibody (bead) in the same manner as done in Reference Example 2.

Reference Example 5

Preparation of labeled antibody

A monoclonal antibody FH-7 (100 µg) was dissolved in 0.5 ml of 0.1M borate buffer (pH 8.0). One mCi of $Na^{125}I$ (product of New England Nuclear Co., Ltd., U.S.A.) was added to the solution.

A dichloromethane solution (40 µl) of 1 mg/ml of Iodogen (product of Pierce Co., Ltd.) was placed into a glass test tube, which was then dried by removing the solvent in a nitrogen gas stream. The antibody solution obtained above was placed into the test tube and then gently stirred with ice cooling for 5 minutes for reaction. The reaction product was placed into another test tube to terminate the reaction and then subjected to gel filtration (Sepharose CL-6B, eluent: 50 mM phosphate buffer containing 0.15M NaCl, 0.1% BSA and 0.02% $NaN_3$), whereby IgG fractions matching in radioactivity peak were collected to obtain $^{125}I$-labeled antibody.

Reference Example 6

Test for immunoreactivity

One NCA bead or 1 NFA bead obtained in Reference Example 2 was added to 200 µl of a solution (about 50,000 cpm) of the labeled antibody obtained in Reference Example 5 in 40 mM sodium citrate buffer (pH 5.0) containing 0.1% BSA and 0.05% $NaN_3$ and the mixture was incubated at room temperature with gentle shaking for 90 minutes.

The bead was washed twice with distilled water and the radioactivity of the bead was counted with a gamma-counter. Fig. 1 shows the result.

In Fig. 1, the radioactivity (B ; cpm) of the bead is plotted as ordinate, and a vertical column at A on the abscissa shows the result given with NCA, a vertical column at B thereon the result afforded with NFA and a vertical column at C the result imparted with the bead obtained with 20 µg/ml solution of HSA (human serum albumin; product of Sigma Chemical Co., Ltd., U.S.A.) as the control in the same manner as done in Reference Example 2.

Fig. 1 shows that NCA and NFA are both useful as the antigens in the immunoassay for determining Disialosyl $Le^a$ antigen.

Example 1

To 10 µl of NCA standard antigen obtained in Reference Example 3 were added a solution (about 70,000 cpm) of the labeled antibody given in Reference Example 5 (in 200 µl of 40 mM sodium citrate buffer (pH 5.0) containing 0.1% BSA, 0.05% $NaN_3$ and 0.1% mouse serum) and one bead of NCA obtained in Reference Example 2. The mixture was incubated with gentle shaking at room temperature for 90 minutes.

Thereafter the bead was washed twice with distilled water and the radioactivity of the bead was counted with a gamma-counter.

Fig. 2 shows the result obtained (standard curve).

In Fig. 2, the concentration (U/ml) of the antigen used is plotted as abscissa and $B/B_0$ (%) [B = radioactivity (cpm) of the bead; $B_0$ = radioactivity (cpm) of the bead when using a blank solution (standard antigen-free solution)] as ordinate.

Fig. 2 reveals that the use of the above determination system results in the determination of Disialosyl $Le^a$ antigen with high precision (duplicate matched) achieved in a short time.

The same result was obtained when NFA (bead) was used. The amounts of Disialosyl $Le^a$ antigens determined with two kinds of standard antigens prepared in Reference Example 3 were substantially equal.

## Example 2

To 10 µl of NCA standard antigen obtained in Reference Example 3 were added 200 µl of 40 mM sodium citrate buffer (pH 5.0) containing 0.1% BSA, 0.05% $NaN_3$ and 2.5% sheep serum and one insolubilized antibody bead obtained in Reference Example 4. The mixture was incubated at room temperature with gentle shaking for 2 hours.

The bead was washed with distilled water twice and 200 µl of the same labeled antibody solution (about 110,000 cpm) as used in Example 1 was added thereto. The mixture was incubated under the same conditions as above. The bead was washed twice in the same manner as above and the radioactivity of the bead was counted with a gamma-counter.

The result obtained (standard curve) is shown in Fig. 3 in which the concentration of the antigen (U/ml) is plotted as abscissa and $B-B_0$ (cpm) as ordinate.

The comparison of Fig. 3 with Fig. 2 shows that the determination system (determination system of Example 1) using the antigen of the invention as an insolubilized one in the immunoassay for determining Disialosyl $Le^a$ antigen can better utilize the characteristics of the antigen of the invention, hence desirable.

## Example 3

Using serums obtained in the usual manner from cancer patients and healthy volunteers as samples in place of standard antigens, the procedures of Examples 1 and 2 were each carried out to determine the amount of Disialosyl $Le^a$ antigen in the samples.

The results obtained were applied to each of the standard curves of Figs. 2 and 3 to give the amount of antigen (amount of Disialosyl $Le^a$ antigen, U/ml) in the sample.

The results obtained in respect of 31 healthy volunteers are indicated below in Table 1.

Table 1

| Determination method | Conc. of antigen (U/ml) | |
|---|---|---|
| | Average | S.D. |
| Method of Ex. 1 | 9.32 | 4.55 |
| Method of Ex. 2 | 19.61 | 9.45 |

The value exceeding a specific level (average + 2 × S.D.) of healthy volunteers (18.42 in the case of method of Ex. 1, 38.69 in the case of method of Ex. 2) was evaluated as positive and the value below that level as negative. The number of cancer patients rated as positive and the number thereof rated as negative were counted. The results are shown below in Table 2.

Table 2

| | Method of Ex. 2 | |
|---|---|---|
| | No. with positive | No. of with negative |
| Method of Ex. 1 | | |
| No. with positive | 30 | 16 |
| No. with negative | 2 | 22 |

Table 2 shows that 16 cases identified as negative in the case of the method of Example 2 are in the group of positive in the case of the method of Example 1 while only 2 cases are reversely evaluated (namely 2 cases identified as negative in the method of Example 1 are in the group of positive in the method of of Example 2). This result confirmed that the method of Example 1 is improved over the method of Example 2 in terms of screening and diagnosing cancers.

Example 4

An acid-soluble fraction prepared in the same manner as in Reference Example 1 (before lyophilization) was placed on a column (44 mm in diameter and 750 mm in length) of Sepharose CL-6B (product of Pharmacia Fine Chemicals) using a saline as an eluent, giving a void fraction which is reactive with the antibody FH-7.

An insolubilized antigen was prepared by the same procedure as in Reference Example 2 with the exception of using the antigen obtained above and a polystyrene bead of 6.5 mm diameter.

The determination of Disialosyl $Le^a$ antigen using the insolubilized antigen (bead) was conducted according to the method described above in Example 1. Stated more specifically, one bead of the insolubilized antigen and a solution (about 10,000 cpm) of labeled antibody [200 μl of 50 mM sodium citrate buffer (pH 5.0) containing 25% sheep serum, 1% mouse serum and 0.05% $NaN_3$] were added to 50 μl of each NCA standard antigen [prepared using seep serum in the same manner as in Reference Example 3 and each having varying concentrations of 0 (blank), 5, 10, 20, 40, 80 and 160 U/ml in terms of optionally predetermined unit (U)]. The mixture was incubated with shaking at room temperature for 90 minutes. The same procedure was repeated with the result shown in Fig. 4 as standard curve in which the concentration of the antigen is plotted as abscissa (U/ml: log dose) and the binding rate as ordinate. The binding rate is determined by the following equation:

$$\text{logit}(\log \frac{(B-BL)/(Bo-BL)}{1-(B-BL)/(B-BL)})$$

wherein B and Bo are as defined hereinbefore, BL is the radioactivity (cpm) of the bead when using a blank solution [sheep serum containing 2% antibody F-H (ascites)].

The foregoing determination by competition method is known theoretically as being able to achieve primary regression due to log logit regression. Fig. 4 shows that the determination of the present invention can be done with extremely high precision [$\gamma$ = -0.9963 (t = -23.245)].

**Claims**

1. An immunoassay for determining Disialosyl $Le^a$ antigen, characterized by using an acid-soluble fraction of excrement as the antigen in the determination system.
2. An immunoassay according to claim 1 wherein the antibody FH-7 is used.
3. An immunoassay according to claim 1 wherein the excrement is meconium.

4. An immunoassay according to claim 1 wherein the acid-soluble fraction of excrement is a perchloric acid-soluble fraction thereof.

5. An immunoassay according to claim 1 wherein the acid-soluble fraction of excrement is used as a standard antigen.

6. An immunoassay according to claim 1 wherein the acid-soluble fraction of excrement is used as an insolubilized antigen.

7. An immunoassay according to claim 6 which is a competitive inhibition assay using the antibody FH-7 in a labeled form.

8. An assay kit for determining Disialosyl $Le^a$ antigen, the kit containing an antigen reagent comprising an acid-soluble fraction of excrement.

9. An assay kit according to claim 8 wherein the excrement is meconium.

10. An assay kit according to claim 8 which contains an antibody recognizing Disialosyl $Le^a$ antigen.

11. An assay kit according to claim 10 wherein the antibody is the antibody FH-7.

12. An assay kit according to claim 8 which comprises (a) an antigen reagent in an insolubilized form and (b) the antibody FH-7 in a labeled form.

13. Use of an acid-soluble fraction of excrement for preparing a pharmaceutical composition for diagnosing cancers.

FIG. 1

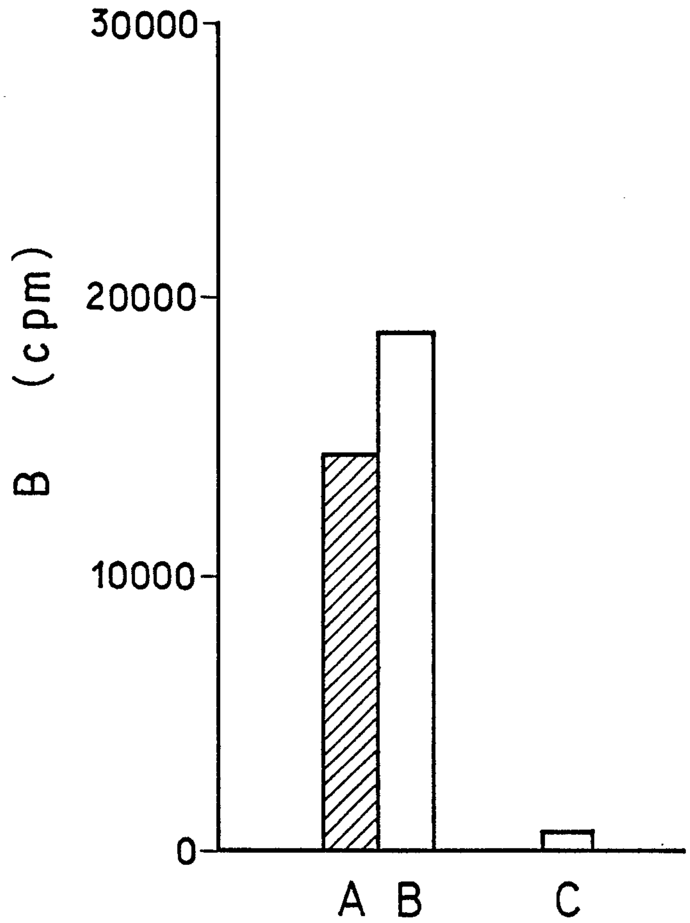

FIG. 2

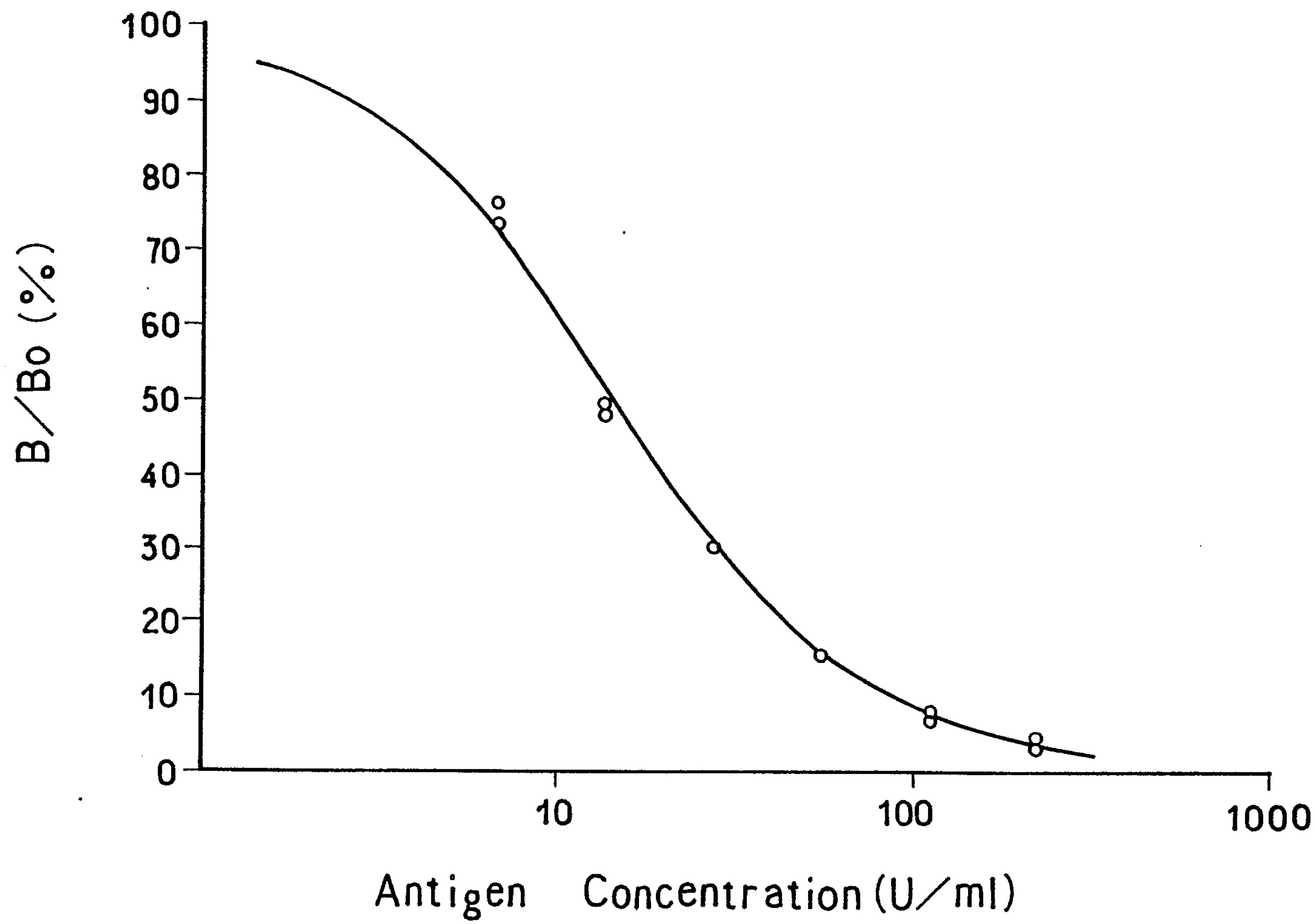
100
90
80
70
60
50
40
30
20
10
0
B/Bo (%)
10
100
1000
Antigen Concentration (U/ml)

FIG. 3

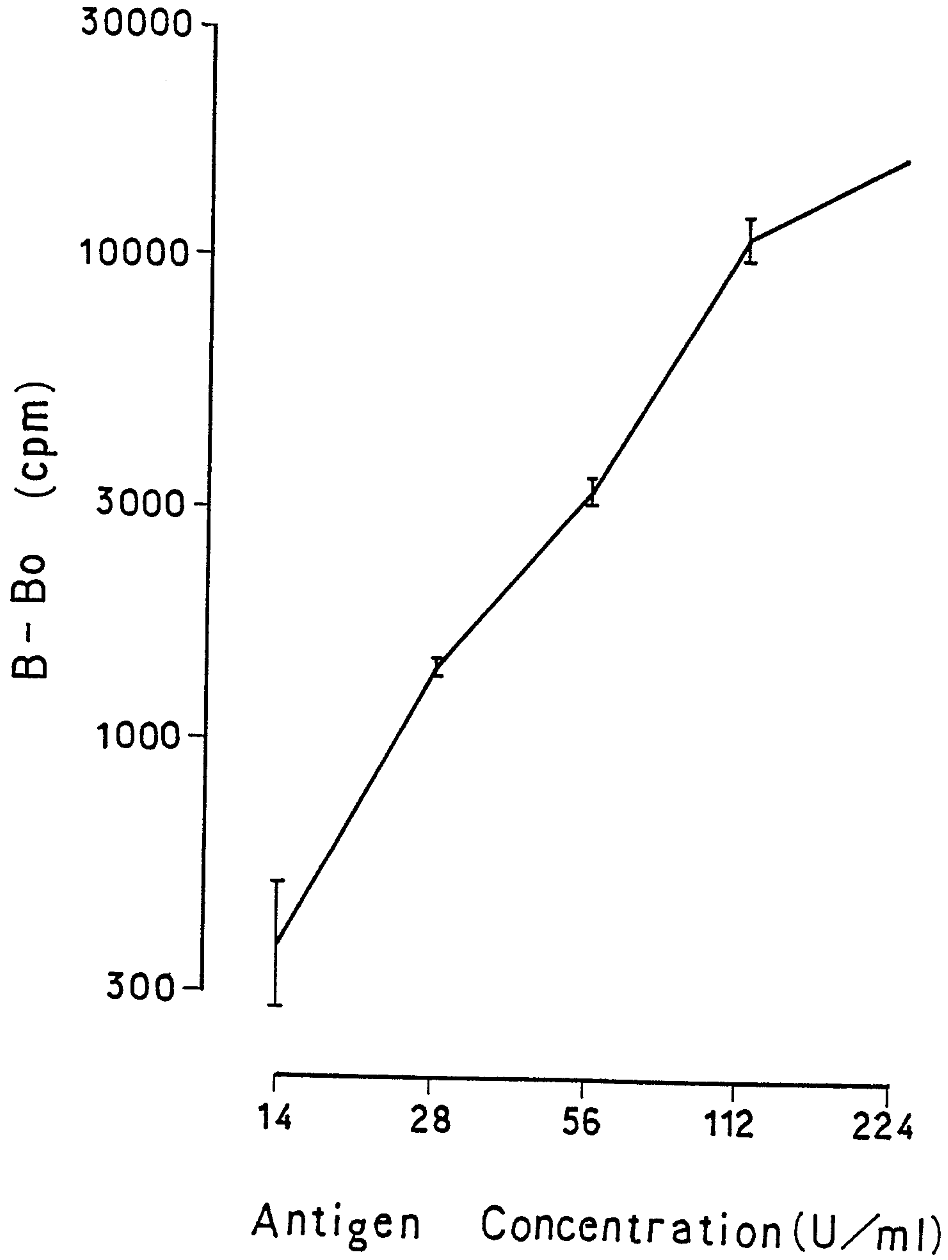
30000
10000
3000
1000
300
B - Bo (cpm)
14
28
56
112
224
Antigen Concentration (U/ml)

FIG. 4

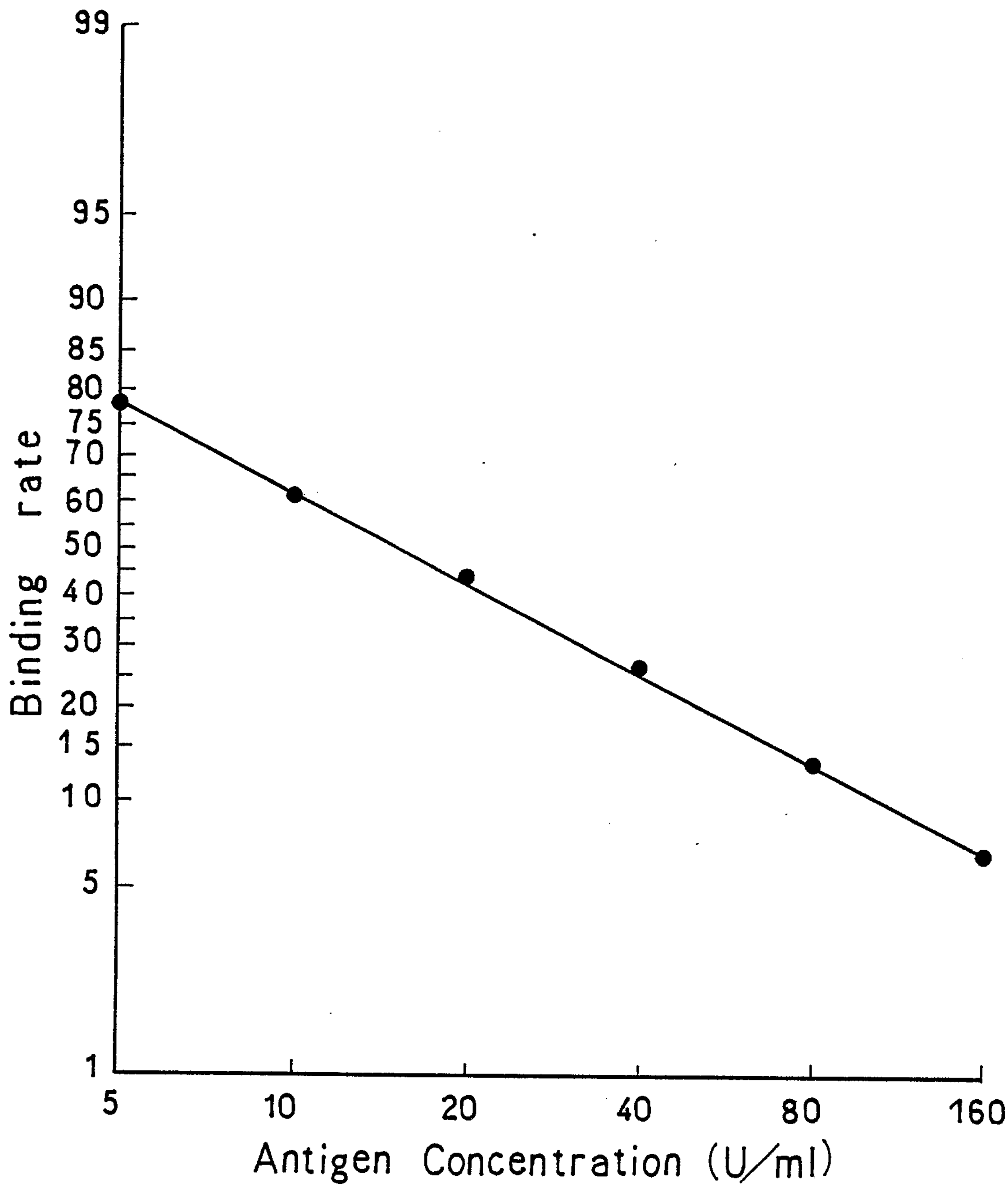
99
95
90
85
80
75
70
60
50
40
30
20
15
10
5
1
Binding rate
5
10
20
40
80
160
Antigen Concentration (U/ml)

European Patent Office

# EUROPEAN SEARCH REPORT

Application number: EP 88105721.0

| Category | DOCUMENTS CONSIDERED TO BE RELEVANT<br>Citation of document with indication, where appropriate, of relevant passages | Relevant to claim | CLASSIFICATION OF THE APPLICATION (Int. Cl 4) |
|---|---|---|---|
| A | EP - A2 - 0 165 811 (THE REGENTS OF THE UNIVERSITY OF CALIFORNIA)<br>* Abstract; claims *<br>-- | 1 | G 01 N 33/574<br>G 01 N 33/577<br>G 01 N 33/531 |
| D,A | THE JOURNAL OF BIOLOGICAL CHEMISTRY, vol. 261, no. 12, April 25, 1986<br>E.NUDELMAN et al. "Novel Fucolipids of Human Adenocarcinoma: Disialosyl $Le^a$ Antigen ($III^4$ $FucIII^6$ $NeuAcIV^3$--$NeuAcLc_4$) of Human Colonic Adenocarcinoma and the Monoclonal Antibody (FH7) Defining This Structure"<br>pages 5487-5495<br>* Abstract *<br>-- | 2 | |
| P,A | EP - A2 - 0 248 147 (SLOAN-KETTERING INSTITUTE FOR CANCER RESEARCH)<br>* Claims 1,22,31,42 *<br>-- | 1 | TECHNICAL FIELDS SEARCHED (Int. Cl.4) |
| A | EP - A2 - 0 173 663 (HOMGREN et al.)<br>* Claims 1-5 *<br>-- | 1 | G 01 N 33/00<br>A 61 K 39/00 |
| A | US - A - 4 507 391 (PUKEL et al.)<br>* Claims *<br>---- | 1 | |

The present search report has been drawn up for all claims

| Place of search | Date of completion of the search | Examiner |
|---|---|---|
| VIENNA | 30-06-1988 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82